# EUROPEAN PATENT SPECIFICATION

(11) **EP 4 201 300 B1**
(45) Date of publication and mention of the grant of the patent: **03.12.2025**
(21) Application number: 22216151.5
(22) Date of filing: 22.12.2022
(51) Int. Cl.: A61B 1/00, A61B 1/05

(54) **MEDICAL SCOPE**
MEDIZINISCHES ENDOSKOP
ENDOSCOPE MÉDICAL

(30) Priority: 22.12.2021 DE 102021134433
(43) Date of publication of application: 28.06.2023
(73) Proprietor: Karl Storz SE & Co. KG, 78532 Tuttlingen (DE)
(72) Inventor: Schwarz, Peter, 78532 Tuttlingen (DE)
(74) Representative: Wimmer, Stephan

(56) References cited:
- EP-A1- 3 769 658
- US-A1- 2006 114 986
- US-A1- 2015 305 602
- US-A1- 2015 359 420

## Description

The present invention refers to a medical scope, in particular a medical endoscope or exoscope or a surgical microscope. In particular, the present invention refers to the transfer of power and a control signal to an image sensor and the transfer of an image signal from the image sensor to a camera control unit, primarily when the image sensor is located at the distal end of the shaft of the medical scope.

An image sensor capturing images requires electrical power. Furthermore, usually a control signal controlling the clock, the electronic shutter, the sensitivity and/or other parameters is provided to the image sensor. An image signal representing images captured by the image sensor is provided by the image sensor and is transferred to a camera control unit or another device processing, analyzing, storing and/or forwarding the image signal to a display. The transfer of power, a control signal and an image signal can be challenging, in particular when the image sensor is located in a small volume distal end of a medical scope and the transfer needs to be along a long and thin shaft. Pivotability or rotatability of the image sensor further increases the technical challenge.

In US 2006/0114986 A1, an adapter for use with digital imaging medical device is described. system includes an endoscope with an image sensor located in a distal tip that is incommunication with an imaging adapter that has image processing system. A data stream output by an image sensor is serialized. A proximal connector connects the endoscope to a corresponding connector on an imaging adapter. The imaging adapter includes a camera card, the camera card containing circuitry for deserialization.

According to US 2015/0305602 A1, a camera body contains a data serializer, and a console includes a data deserializer.

In EP 3 769 658 A1, a scope for examining or surgically treating ears is described. The scope comprises a probe and an eyball-like camera rotatably connected to the probe at axis pins. One power pin contact is delivered through each axis pin so that power is delivered through these pin contacts and data from the camera is delivered using a wireless technology. A cable from a wireless communicator delivers data to an image processor outside of the scope.

In US 2015/0359420, an endoscope with imaging unit at a tip end portion of an insertion section is described. The imaging unit has a board on which the image pickup device is installed and a cable through which a signal is input to and output from the board. The imaging unit is rotatably attached to support arm in the inside of a hard part at the tip end portion of the insertion section. The imaging unit is able to rotate around an axis orthogonal to a longitudinal axis of the insertion section. The imaging unit has a board and an image pickup device is installed on one surface of the board. One end of a cable for performing input and output of a signal with respect to the board is assembled on and fixed to the other surface of board. The cable comprises a plurality of coaxial cables disposed in a belt shape.

An object of the present invention is to provide an improved medical scope.

This object is solved by the subject matter of the independent claim.

Further embodiments are described in the dependent claims.

A medical scope according to the present invention as defined in independent claim 1 comprises an objective producing an image of an object, an image sensor capturing the image produced by the objective and providing an image signal representing the captured image, a serializer comprising a two-conductor first interface and a multi-conductor second interface, the multi-conductor second interface is connected to the image sensor, a first sliding contact and a second sliding contact connected to the two-conductor first interface of the serializer, a third sliding contact in electrically conductive contact to the first sliding contact and a fourth sliding contact in electrically conductive contact to the second sliding contact, wherein at least two of the first sliding contact, the second sliding contact, the third sliding contact and the fourth sliding contact are coaxial.

A preferred embodiment further comprises a deserializer comprising a two-conductor first interface coupled to the two-conductor first interface of the serializer by a pair of conductors and a multi-conductor second interface coupled to a camera control unit or to a connector which can be connected to a camera control unit.

The medical scope is in particular an endoscope or an exoscope or a surgical microscope. The objective comprises one or more lenses. The objective can provide a fixed focal length or a variable focal length. The objective and the image sensor can be in a fixed-focus configuration. As an alternative, the objective can be controllable to produce sharp images of objects within a range of distances.

The serializer is configured to receive electrical power and optionally also a control signal via its two-conductor (or two-wire or two-core) first interface and provide the power and optionally the control signal at two or more conductors of the multi-conductor (or multi-wire or multi-core) second interface and to receive the image signal provided by the image sensor via two or more conductors of the multi-conductor second interface and transmit the image signal via the two-conductor first interface.

The deserializer is configured to receive electrical power and optionally also a control signal from a camera control unit or any other device via two or more conductors of its multi-conductor second interface and to transmit the power and optionally also the control signal via its two-conductor first interface to the two-conductor first interface of the serializer. Furthermore, the deserializer is configured to receive via its two-conductor first interface the image signal and to transmit the image signal via its multi-conductor second interface to a camera control unit.

According to the invention, both power and optionally the control signal and an image signal are transferred (in opposite directions) via the same two-conductor connection. This two-conductor connection requires less space than a multi-conductor connection and facilitates new technical solutions to challenges like pivotability or rotatability of the image sensor or separability of components of the medical scope.

A medical scope comprising a deserializer as it is described herein in particular further comprises a first sliding contact and a second sliding contact connected to the two-conductor first interface of the serializer and a third sliding contact and a fourth sliding contact coupled to the two-conductor first interface of the deserializer, wherein the first sliding contact is in electrically conductive contact to the third sliding contact and the second sliding contact is in electrically conductive contact to the fourth sliding contact, and wherein at least two of the first sliding contact, the second sliding contact, the third sliding contact and the fourth sliding contact are coaxial.

Each one of the first and second sliding contacts is connected to a respective conductor of the two-conductor first interface of the serializer. Each one of the third and fourth sliding contacts is (directly or indirectly) coupled to a respective conductor of the two-conductor first interface of the deserializer.

In particular, at least two of the first sliding contact, the second sliding contact, the third sliding contact and the fourth sliding contact are shaped as sections of surfaces of cylinders or cones or other geometrical objects which provide rotational symmetry with respect to the same axis of symmetry. In particular, each of the first sliding contact and the second sliding contact are annular sections of cylinder surfaces rotationally symmetric with respect to the same axis of symmetry, wherein the third and fourth sliding contacts can be coaxial as well or can provide other geometry.

A medical scope as it is described herein in particular further comprises an image sensor unit including the image sensor, the serializer, the first sliding contact and the second sliding contact and a shaft with a distal end zone including the third sliding contact and the fourth sliding contact.

The image sensor unit can be pivotable or rotatable about an axis of rotation relative to the shaft. This axis of rotation is in particular identical to the axis of symmetry of at least two of the first sliding contact, the second sliding contact, the third sliding contact and the fourth sliding contact.

In a medical scope as it is described herein, the first sliding contact and the second sliding contact are in particular part of a first coaxial plug connector, wherein the third sliding contact and the fourth sliding contact are part of a second coaxial plug connector complementary to the first coaxial plug connector.

The first coaxial plug connector is in particular part of or mechanically integrated with the image sensor unit. The second coaxial plug connector is in particular part of or mechanically integrated with the distal end zone of the shaft. The complementary coaxial plug connectors can allow the image sensor unit to be separable from the shaft. Separability can facilitate cleaning and sterilization as well as replacement of the image sensor unit in case of failure or malfunction. Furthermore, the image sensor unit can be easily replaced if other characteristics are required, for example regarding resolution, sensitivity, field of view or spectral sensitivity.

In a medical scope as it is described herein, an axis of symmetry of the coaxial sliding contacts is in particular parallel to the viewing direction of the image sensor unit.

In particular in case of a stereoscopic medical scope this allows the stereo base to be rotated and, in particular, to be held horizontal.

In a medical scope as it is described herein, the axis of symmetry of the coaxial sliding contact is in particular orthogonal to the viewing direction of the image sensor unit and orthogonal to the longitudinal axis of the distal end zone of the shaft.

Two axes or directions are orthogonal if they include an angle between 80 degrees and 100 degrees or between 85 degrees and 95 degrees or if the included angle is essentially or exactly 90 degrees.

This orientation of the axis of symmetry allows a largely free rotation of the viewing direction.

A medical scope as it is described herein in particular further comprises a fifth sliding contact connected to the third sliding contact, a sixth sliding contact connected to the fourth sliding contact, a seventh sliding contact in electrically conductive contact to the fifth sliding contact and an eighth sliding contact in electrically conductive contact to the sixth sliding contact, wherein at least two of the fifth sliding contact, the sixth sliding contact, the seventh sliding contact and the eighth sliding contact are coaxial.

In particular, a pair of conductors connects the fifth sliding contact to the third sliding contact and the sixth sliding contact to the fourth sliding contact, respectively. In particular, another pair of conductors connects the seventh sliding contact and the eighth sliding contact to the two-conductor first interface of the deserializer.

In particular, at least two of the fifth sliding contact, the sixth sliding contact, the seventh sliding contact and the eighth sliding contact are shaped as sections of surfaces of cylinders or cones or other geometrical objects which provide rotational symmetry with respect to the same axis of symmetry. In particular, each of the fifth sliding contact and the sixth sliding contact are annular sections of cylinder surfaces rotationally symmetric with respect to the same axis of symmetry, wherein the third and fourth sliding contacts can be coaxial as well or can provide other geometry.

The image sensor or, if applicable, the image sensor unit or part of the image sensor unit can be pivotable or rotatable about a second axis of rotation relative to the shaft. This second axis of rotation is in particular identical to the axis of symmetry of at least two of the fifth sliding contact, the sixth sliding contact, the seventh sliding contact and the eighth sliding contact.

In particular, the fifth sliding contact and the sixth sliding contact are part of a third coaxial plug connector and the seventh sliding contact and the eighth sliding contact are part of the fourth coaxial plug connector complementary to the third coaxial plug connector. In this case, the fifth, sixth, seventh and eighth sliding contacts can provide a second separable plug connection and/or a second axis of rotation the image sensor or the image sensor unit can be rotated or pivoted about.

In a medical scope as it is described herein, an axis of symmetry of at least two of the first sliding contact, the second sliding contact, the third sliding contact and the fourth sliding contact and an axis of symmetry of at least two of the fifth sliding contact, the sixth sliding contact, the seventh sliding contact and the eighth sliding contact enclose an angle greater than zero.

In particular, the axes of symmetry enclose an angle between 70 degrees and 110 degrees or between 80 degrees and 100 degrees or essentially or exactly 90 degrees.

In particular in case of a stereoscopic medical scope, these two orthogonal axes of symmetry can be axes of rotation and can facilitate a free or largely free rotation of the viewing direction while the stereo base can be held horizontal continuously.

In a medical scope as it is described herein, the serializer and the deserializer in particular comply with the CSI camera serial interface specification of the MIPI mobile industry processor interface alliance.

The CSI specification of the MIPI alliance provides a well-established, verified and robust standard ensuring high performance and reliability.

The serializer can be integrated with the image sensor. In particular the serializer and the image sensor can be located on or in the same die, chip or device. The deserializer can be located close to or can even be partially or fully integrated with a camera control unit, wherein the camera control unit can be partially or fully integrated with the medical scope or can be a separate device. When the deserializer is part of the medical scope it is in particular located in or at the proximal end of the medical scope.

In a medical scope as it is described herein, the serializer and the deserializer are in particular FPD modules.

FPD modules provide the option to establish an FPD-Link (including FPD-Link II, FPD-Link III and further improved versions. FPD-Link (Flat Panel Display Link) is a free and open standard for connecting the output of a graphics processing unit to a display panel's timing controller and was created in 1996 by National Semiconductor. For example, FPD-Link II uses only one twisted pair for the transmission of both 24-bit color video data and a clock signal. FPD-Link III embeds a bidirectional communication channel on the same differential pair of conductors. For example, each of the serializer and the deserializer is or comprises a device DS90UB953-Q1 by Texas Instruments or a similar device.

A medical scope as it is described herein is in particular a stereoscopic endoscope or exoscope or surgical microscope capturing a first image to be displayed to the left eye of medical personnel and a second image to be displayed to the right eye of the medical personnel. The first and second images can be captured simultaneously or almost simultaneously, for example alternatingly.

### Short description of the figures

Embodiments will be described below with reference to the enclosed figures.
- Figure 1: shows a schematic representation of an endoscope;
- Figure 2: shows a schematic representation of another endoscope;
- Figure 3: shows a schematic representation of another endoscope;
- Figure 4: shows a magnified schematic representation of the distal end zone of the shaft of one of the endoscopes shown in Figures 1 through 3;
- Figure 5: shows another schematic representation of the distal end zone of Figure 4;
- Figure 6: shows a schematic representation of another distal end zone,
- Figure 7: shows a schematic representation of another distal end zone,
- Figure 8: shows a schematic representation of another distal end zone.

### Description of embodiments

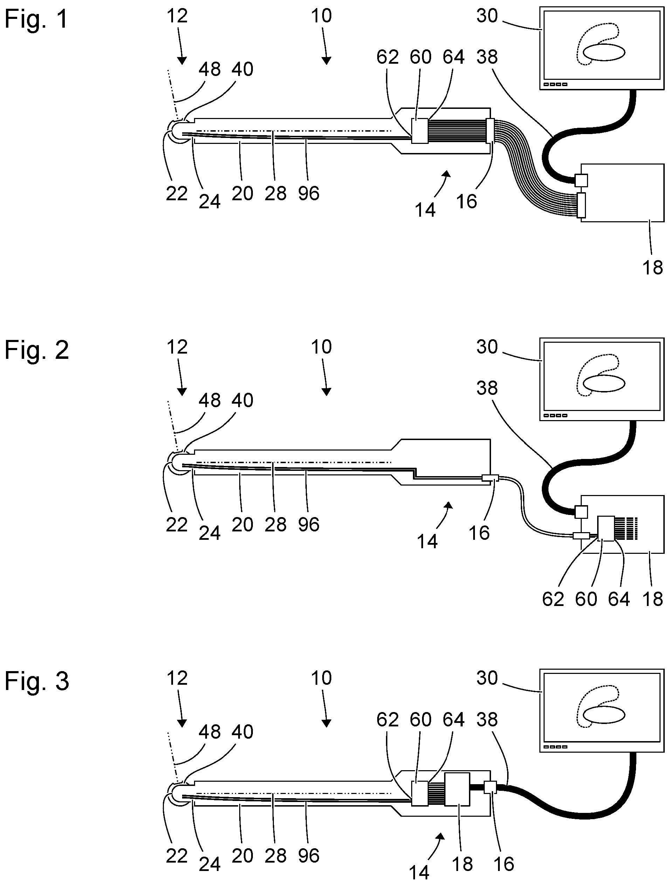

Figure 1 shows a schematic representation of an endoscope 10 as an example of a medical scope. The endoscope 10 provides a distal end 12 to be inserted into a natural or artificial cavity in the body of a human or animal patient and a proximal end 14. The proximal end 14 can provide a user interface, for example one or more push buttons, sliders, rotatable knobs which can be manipulated by one or more fingers and control mechanical, electrical, hydraulic, pneumatic or other functions of the endoscope. Furthermore, the proximal end 14 of the endoscope 10 provides a connector 16 which can be connected to a camera control unit 18 by a cable.

The endoscope 10 provides a shaft 20 with a distal end 22, a distal end zone 24 and a longitudinal axis 28. The longitudinal axis 28 of the shaft is in particular defined as the line formed by the center points of all cross sections of the shaft 20.

In the example shown in Figure 1, the shaft 20 is straight and in particular rigid. As an alternative, parts of the shaft 20 or the entire shaft can be bended or flexible.

An image sensor unit 40 is connected to the distal end zone 24 of the shaft 20. The image sensor unit 40 includes one or more objectives or lenses producing one or more images and one or more image sensors capturing the image or the images. The arrangement of at least one objective and the corresponding image sensor defines the viewing direction 48 of the image sensor unit 40. In the example shown, the image sensor unit 40 and the viewing direction 48 can be pivoted about an axis orthogonal to the drawing plane of Figure 1.

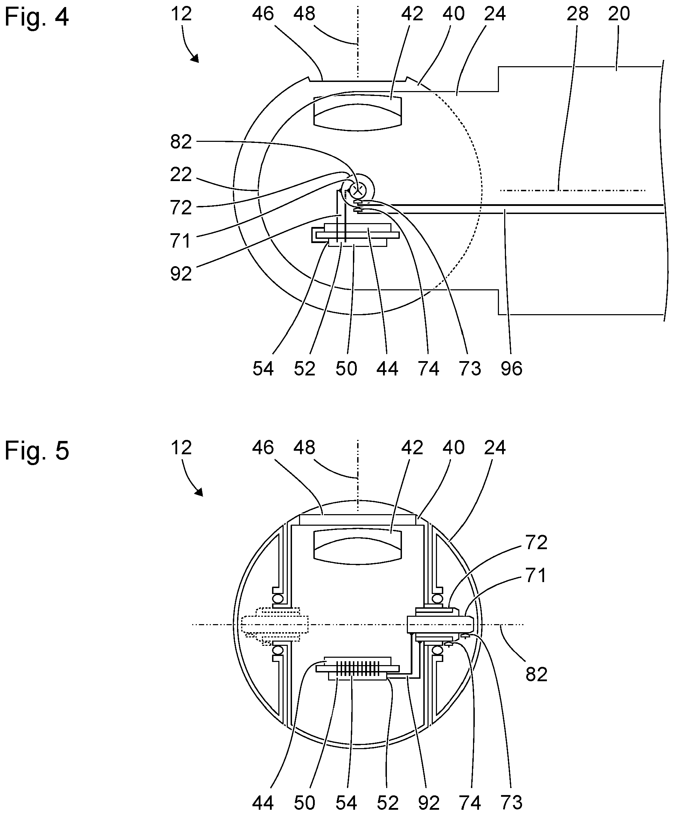

From a functional point of view, the image sensor unit 40 can be seen as a part of the shaft 20 providing the very distal end 12 of the endoscope 10. From the design perspective or from the perspective of mechanical structure, the shaft 20 and the image sensor unit 40 are two modules or members mechanically coupled together. More details of the distal end zone 24 of the shaft 20 and the image sensor unit 40 are described with reference to Figures 4 through 7.

In the shaft 20, a pair of conductors 96 connects a serializer located in the image sensor unit 40 to a deserializer 60 located in the proximal end 14 of the endoscope 10. The deserializer 60 provides a two-conductor first interface 62 connected to a corresponding two-conductor first interface of the serializer in the image sensor unit 40 and a multi-conductor second interface 64 connected to the camera control unit 18 via the connector 16.

The deserializer 60 receives at its multi-conductor second interface 64 electrical power and a control signal from the camera control unit 18 and transmits electrical power and the control signal via its two-conductor first interface 62 and the pair of conductors 96 to the serializer in the image sensor unit 40. Furthermore, the deserializer 60 receives at its two-conductor first interface 62 an image signal transmitted by the serializer in the image sensor unit 40 via the pair of conductors 96. The deserializer 60 forwards this image signal through its multi-conductor second interface 64 and via the connector 16 to the camera control unit 18.

Thus, both electrical power and the control signal are provided to the image sensor unit 40 and the image signal is received from the image sensor unit 40 via the same single pair of conductors 96.

The camera control unit provides electrical power and the control signal, receives and processes the image signal and provides the processed image signal to a display device 30 connected to the camera control unit 18 by a cable 38.

Figure 2 shows a schematic representation of another endoscope 10 similar to the endoscope described above with reference to Figure 1 with respect to many features, characteristics and functions. Differences between the endoscope 10 shown in Figure 2 and the endoscope described above with reference to Figure 1 are described below.

The endoscope 10 shown in Figure 2 does not comprise a deserializer. Rather, the camera control unit 18 comprises a deserializer 60 similar to the deserializer of the endoscope described above with reference to Figure 1. Thus, the connector 16 at the proximal end 14 of the endoscope 10 and the cable connecting the connector 16 to the camera control unit 18 can be of the two-conductor type. The two-conductor first interface 62 of the deserializer 60 is connected to the connector 16 of the endoscope 10 by a cable. The multi-conductor second interface 64 of the deserializer 60 is connected to further components of the camera control unit 18 not shown in Figure 2.

Figure 3 shows a schematic representation of another endoscope 10 similar to the endoscope described above with reference to Figure 1 with respect to many features, characteristics and functions. Differences between the endoscope 10 shown in Figure 3 and the endoscope described above with reference to Figure 1 are described below.

In the example shown in Figure 3, the camera control unit 18 is integrated into the proximal end 14 of the endoscope 10. The endoscope 10 provides a fully processed and conditioned image signal for a display which can be directly coupled to the connector 16. In this case, the endoscope 10 may receive electrical power from the display via the connector 16.

Figure 4 shows a magnified schematic representation of the shaft 20, the distal end zone 24 of the shaft and the image sensor unit 40 forming the very distal end 12 of one of the endoscopes described above with reference to Figures 1 through 3. The distal end zone 24 of the shaft 20 is bifurcated. In the viewing direction of Figure 4, one prong is in front of the image sensor unit 40, and the other prong is behind the image sensor unit 40. The contour of the image sensor unit 40 is shown in a broken line where it is actually hided behind one prong of the distal end zone 24 of the shaft 20. The essentially circular contour of the image sensor unit 40 is visible.

A flat area 46 of the outer surface of the image sensor unit 40 is formed by a light entrance window. In the example shown in Figure 1, the flat area 46 provides the only deviation from a circular contour.

In Figure 4, components located inside the visible prong of the distal end zone 24 or inside the image sensor unit 40 and, thus, actually invisible in reality, are shown. In particular, the image sensor unit 40 comprises an objective 42 producing a real image and an image sensor 44 capturing the real image produced by the objective 42 and providing an image signal representing the captured image. The arrangement of the objective 42 and the image sensor 44 constitutes the viewing direction 48.

Furthermore, the image sensor unit 40 comprises a serializer 50 with a two-conductor first interface 52 and a multi-conductor second interface 54. The multi-conductor second interface 54 is connected to the image sensor 44 by a corresponding number of parallel conductors. A pair of conductors 92 connects the two-conductor first interface 52 of the serializer 50 to a first sliding contact 71 and a second sliding contact 72, respectively. Each of the first sliding contact 71 and the second sliding contact 72 is cylindrical with a circular cross section and identical cylinder axes 82 orthogonal to the drawing plane of Figure 4. Thus, both the first and the second sliding contact 71, 72 provide rotational symmetry with respect to the same axis of symmetry 82.

The first and second sliding contacts 71, 72 are arranged coaxial are rigidly connected to or integral with other components of the image sensor unit 40, in particular with the housing of the image sensor unit 40. The image sensor unit 40 including the first and second sliding contacts 71, 72 can be rotated about the axis 82 of symmetry of the first and second sliding contacts 71, 72.

As will be described in more detail in Figure 5, the first and second sliding contacts 71, 72 of the image sensor unit 40 protrude from the overall outer surface of the image sensor unit 40 and engage in a recess in the prong of the distal end zone 24. At the inner surface of their recess, one or more third sliding contacts 73 in electrically conductive contact to the first sliding contact 71 and one or more fourth sliding contacts 74 in electrically conductive contact to the second sliding contact 72 are arranged. The pair of conductors 96 connects the one or more third sliding contacts 73 and the one or more fourth sliding contacts 74, respectively, to the two-conductor first interface 62 of the deserializer 60 (cf. Figures 1 through 3).

As a result, the image sensor 44 is connected to the camera control unit 18 by the serializer 50, the pair of conductors 92, the first and second sliding contacts 71, 72 rigidly connected to the rest of the image sensor unit 40, the third and fourth sliding contacts 73, 74 rigidly connected to the distal end zone 24 of the shaft 20, the pair of conductors 96 and the deserializer 60 shown in Figures 1 throuth 3. While electrical power and the control signal (from the camera control unit 18 to the image sensor 44) and the image signal representing the captured image (from the image sensor 44 to the camera control unit 18) are usually transferred via different conductors or sets of conductors between the camera control unit 18 and the deserializer 60 and between the serializer 50 and the image sensor 44, both electrical power and the control signal and the image signal are transferred via the same pair of conductors 92, via one coaxial arrangement of sliding contacts 71, 72, 73, 74 and again a pair of conductors 96 between the serializer 50 and the deserializer 60. Thus, the room or space required for the transfer of electrical power, the control signal and the image signal, in particular between the distal end zone 24 of the shaft 20 and the image sensor unit 40 is considerably reduced.

Figure 5 shows another schematic representation of the endoscope's distal end 12 described above with reference to Figure 4. The drawing plane of Figure 5 is orthogonal to the drawing plane of Figure 4 and orthogonal to the longitudinal axis 28 of the shaft 20 (cf. Figures 1 through 4) and comprises the axis of symmetry 82 of the first and second sliding contacts 71, 72 about which the image sensor unit 40 can be rotated. Again, both the contours of the prongs forming the distal end zone 24 of the shaft and the contour of the image sensor unit 40 and components located inside the prongs forming the distal end zone 24 of the shaft 20 and components located inside the image sensor unit 40 are shown in Figure 5.

The first sliding contact 71 is formed by a circular cylindrical and electrically conductive rod. The second sliding contact 72 is formed by an electrically conductive tube arranged coaxial to the rod. Isolating structures (for example made from ceramics or plastics) electrically insulate the first and second sliding contacts 71, 72 from each other and optionally also from the housing of the image sensor unit 40. A sealing ring can prevent entry of a fluid or other debris into the recess in which the third and fourth sliding contacts 73, 74 are arranged.

In the example shown in Figures 4 and 5, the image sensor 44 and the serializer 50 are distinct (and different) components arranged at opposite sides of a circuit board. As an alternative, the image sensor 44 and the serializer 50 can be arranged at the same side of a circuit board or in any other geometrical configuration or can be integrated in one chip or even in one die.

In the example shown in Figures 4 and 5, the first and second sliding contacts 71, 72 protrude from the housing of the image sensor unit 40 and engage into a recess in the distal end zone 24 of the shaft where the third and fourth sliding contacts 73, 74 are arranged. As an alternative, the third and fourth sliding contacts 73, 74 can protrude from the distal end zone 24 and engage into a recess in the image sensor unit 40.

In the example shown in Figures 4 and 5, only the first and second sliding contacts 71, 72 provide rotational symmetry with respect to the axis 82. As an alternative, both the first and second sliding contacts 71, 72 and the third and fourth sliding contacts 73, 74 can provide rotational symmetry with respect to the axis 82. As a further alternative, only the third and fourth sliding contacts 73, 74 provide rotational symmetry with respect to the axis 82. As a further alternative, one of the first and third sliding contacts 71, 73 and one of the second and fourth sliding contacts 72, 74 provides rotational symmetry with respect to the axis 82.

In the example shown in Figures 4 and 5, only one coaxial connector formed by the first, second, third and fourth sliding contacts 71, 72, 73, 74 is provided. As an alternative, a second coaxial connector providing rotational symmetry with respect to the same axis 82 can be provided as indicated by broken lines.

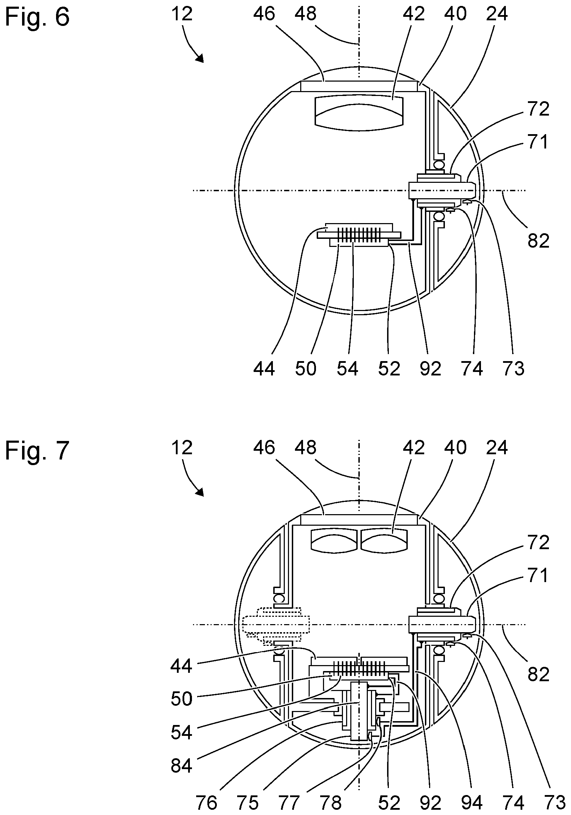

Figure 6 shows a schematic representation of a distal end 12 of an endoscope similar to the endoscopes described above with reference to Figures 1 through 5 with respect to many features, characteristics and functions. Differences between the distal end 12 shown in Figure 6 and the distal end described above with reference to Figures 4 and 5 are described below. The way the distal end 12 of the endoscope is displayed in Figure 6 is similar to Figure 5.

The distal end 12 displayed in Figure 6 differs from the distal end described above with reference to Figures 4 and 5 in that the distal end zone 24 of the shaft is not bifurcated. Rather, the distal end zone is formed by only one rod like structure with a cross section similar to a circular segment. As a consequence, the mechanical connection between the distal end zone 24 and the image sensor unit 40 can be non-destructively separable. In particular, the first and second sliding contacts 71, 72 are part of a first coaxial plug connector rigidly connected to the image sensor unit 40, and the third and fourth sliding contacts 73, 74 are parts of a coaxial plug connector integrated into the distal end zone 24 of the shaft. The image sensor unit 40 can be pulled off from the distal end zone 24 in a direction parallel to the axis of symmetry 82 of at least two of the sliding contacts 71, 72, 73, 74. This can facilitate disassembly before the endoscope is cleaned, replacement of a faulty image sensor unit 40 or replacement of an image sensor unit 40 by an image sensor unit with different characteristics.

Figure 7 shows a schematic representation of a distal end 12 of an endoscope similar to the endoscopes described above with reference to Figures 1 through 5 with respect to many features, characteristics and functions. Differences between the distal end 12 displayed in Figure 7 and the distal end described above with reference to Figures 4 and 5 are described below. The way the distal end 12 is shown in Figure 7 is similar to Figures 5 and 6.

There are two main differences between the distal end 12 shown in Figure 7 and the distal end described above with reference to Figures 4 and 5. One main difference regards stereoscopic imaging, the other main difference regards rotatability about a second axis. Although rotatability about a second axis is particularly relevant in case of stereoscopic imaging, both main differences are independent from one each other to a large extent. Stereoscopic imaging can be realized without rotatability about a second axis, and rotatability about a second axis can be realized in a monocular endoscope as well.

Regarding stereoscopic imaging, two objectives 42 and two image sensors 44 are provided capturing two images from slightly different positions. In the example shown in Figure 7, both image sensors 44 are connected to two distinct multi-conductor second interfaces 54 of the same serializer 50 or to partly or entirely distinct parts of the same multi-conductor second interface 54 of the same serializer 50. As an alternative, two serializers 50 are provided and the multi-conductor second interface 54 of each of the serializers 50 is connected to a respective one of the image sensors 44.

Regarding rotatability about the second axis, the entire arrangement of the two objectives 42 and the two image sensors 44 is rotatable about a second axis 84 orthogonal to the first axis 82 and parallel to or identical with the viewing direction 48 of the image sensor unit 40. The second axis 84 can be pivoted about the first axis 82 together with the image sensor unit 40. In the configuration shown in Figure 7, the drawing plane comprises both the second axis 84 and the viewing direction 48, and the second axis 84 and the viewing direction 48 are orthogonal to the longitudinal axis of the shaft. In particular, the image sensor unit 40 comprises a housing pivotable about the first axis 82, wherein the arrangement formed by the objectives 42, the image sensors 44 and the serializer 50 can be rotated about the second axis 84 within the housing of the image sensor unit 40.

An arrangement of a fifth sliding contact 75, a sixth sliding contact 76, a seventh sliding contact 77 and an eighth sliding contact 78 is similar to the arrangement of the first sliding contact 71, the second sliding contact 72, the third sliding contact 73 and the fourth sliding contact 74. The fifth, sixth, seventh and eighth sliding contact 75, 76, 77, 78 form a two-conductor coaxial connector coupling the pair of conductors 92 coupled to the two-conductor first interface 52 of the serializer 50 and a pair of conductors 94. The other end of the pair of conductors 94 is connected to the first and second sliding contacts 71, 72. Rotational symmetry of at least one of the fifth sliding contact 75 and the seventh sliding contact 77 and of at least one of the sixth sliding contact 76 and the eighth sliding contact 78 about the second axis 84 guarantees a continuous two-conductor connection of the two-conductor first interface 52 of the serializer 50 to the pair of conductors 94. In total, the sliding contacts 71, 72, 73, 74, 75, 76, 77, 78 and their symmetries about the first axis 82 and the second axis 84, respectively, facilitate that the image sensor unit 40 can be freely rotated about the first axis 82 and simultaneously the stereoscopic base defined by the arrangement of the objectives 42 and the image sensors 44 can be freely rotated about the second axis 84. Both power and control signals can be provided to the image sensors 44 and image signals can be received from the image sensors 44 in any angular position of the image sensor unit 40 and in any angular position of the stereoscopic base.

In the example shown in Figure 7, the distal end zone 24 of the shaft is bifurcated. As an alternative, the distal end zone can be embodied similar to the example described above with reference to Figure 6.

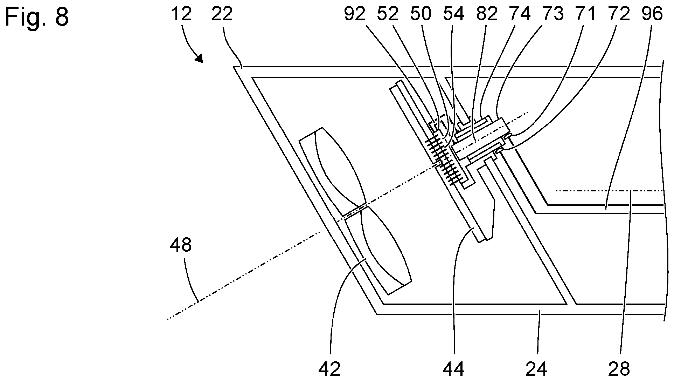

Figure 8 shows a schematic representation of the distal end 12 of another endoscope similar to the endoscopes described above with reference to Figures 1 through 7 with respect to many features, characteristics and functions. Differences between the distal end 12 shown in Figure 8 and the distal ends described above with reference to Figures 4 through 7 are described below.

The endoscope the distal end 12 of which is shown in Figure 8 comprises two objectives 42 and two image sensors 44 providing for stereoscopic imaging. The arrangement formed by the objectives 42 and the image sensors 44 and, thus, the stereoscopic base, can be rotated about an axis 82 parallel to the viewing direction 48. A pair of conductors 92 and an at least partially coaxial arrangement of first, second, third and fourth sliding contacts 71, 72, 73, 74 similar to the arrangements of sliding contacts described above with reference to Figures 4 through 7 provide a two-conductor connection between the two-conductor first interface 52 of the serializer 50 and a pair of conductors 96 running along the shaft 20 in any angular position of the stereoscopic base.

The endoscope the distal end 12 of which is shown in Figure 8 provides a predetermined non-zero angle between the viewing direction 48 and the longitudinal axis 28 of the distal end zone 24 of the shaft 20.

The endoscope the distal end 12 of which is shown in Figure 8 has no image sensor unit distinguishable from the shaft 20. Rather, the objectives 42, the image sensors 44 and the serializer 50 are integrated into the (non-bifurcated) distal end zone 24 of the shaft 20.

While the endoscope the distal end 12 of which is shown in Figure 8 provides stereoscopic imaging, the endoscope can be provided and configured for monocular imaging as well.

While the endoscope the distal end of which is shown in Figure 8 provides a rotation of the objectives 42 and the image sensors 44 about an axis 82 parallel to the viewing direction 48, the axis 82 and the viewing direction 48 may alternatively enclose a non-zero angle.

While the endoscope the distal end 12 of which is shown in Figure 8 provides a predetermined angle between the viewing direction 48 and the longitudinal axis 28 of the distal end zone 24 of the shaft 20, this angle can be variable as well, for example similar to the example described above with reference to Figure 7 comprising a second at least partially coaxial arrangement of sliding contacts.

While the medical scopes described above with reference to Figures 1 through 8 are endoscopes, the present invention can be applied to exoscopes or surgical microscopes as well.

### List of reference numerals

- 10: endoscope
- 12: distal end of the endoscope 10
- 14: proximal end of the endoscope 10
- 16: connector at the proximal end 14 of the endoscope 10
- 18: camera control unit in the proximal end 14 of the endoscope 10 or coupled to the connector 16
- 20: shaft of the endoscope 10
- 22: distal end of the shaft 20
- 24: distal end zone of the shaft 20
- 28: longitudinal axis of the distal end zone 24 of the shaft 20
- 30: display device
- 38: cable connecting the display device 30 to the camera control unit 18
- 40: image sensor unit at the distal end zone 24 of the endoscope 20
- 42: objective of the image sensor unit 40
- 44: image sensor of the image sensor unit 40
- 46: flat area of the outer surface of the image sensor unit 40
- 48: viewing direction of the image sensor 44 and the image sensor unit 40
- 50: serializer in the image sensor unit 40
- 52: two-conductor first interface of the serializer 50
- 54: multi-conductor second interface of the serializer 50
- 60: deserializer at the proximal end 18 of the endoscope 10
- 62: two-conductor first interface of the deserializer 60
- 64: multi-conductor second interface of the deserializer 60
- 71: first sliding contact
- 72: second sliding contact
- 73: third sliding contact
- 74: fourth sliding contact
- 75: fifth sliding contact
- 76: sixth sliding contact
- 77: seventh sliding contact
- 78: eighth sliding contact
- 82: first axis about which the image sensor unit 40 can be pivoted and axis of symmetry of at least two of the first sliding contact 71, the second sliding contact 72, the third sliding contact 73 and the fourth sliding contact 74
- 84: second axis about which the image sensor unit 40 (and the first axis 82) can be rotated and axis of symmetry of at least two of the fifth sliding contact 75, the sixth sliding contact 76, the seventh sliding contact 77 and the eighth sliding contact 78
- 92: pair of conductors connecting the two-conductor interface 52 of the serializer 50 to first and second sliding contacts 71, 72
- 94: pair of conductors connecting the third and fourth sliding contacts 73, 74 or the fifth and sixth sliding contacts 75, 76 to the two-conductor interface 62 of the deserializer 60
- 96: pair of conductors connecting the third and fourth sliding contacts 73, 74 or the seventh and eighth sliding contacts 77, 78 to the two-conductor interface 62 of the deserializer 60

## Claims

1. **Medical scope** (10) comprising:
an **objective** (42) producing an image of an object;
an **image sensor** (44) capturing the image produced by the objective (42) and providing an image signal representing the captured image;
a **serializer** (50) comprising a two-conductor first interface (52) and a multi-conductor second interface (54), said multi-conductor second interface (54) being connected to the image sensor (44);
**characterised in that** the medical scope further comprises:
a first **sliding contact** (71) and a second **sliding contact** (72) connected to the two-conductor first interface (52) of the serializer (50);
a third **sliding** contact (73) in electrically conductive contact to the first sliding contact (71) and a fourth **sliding contact** (74) in electrically conductive contact to the second sliding contact (72),
wherein at least two of the first sliding contact (71), the second sliding contact (72), the third sliding contact (73) and the fourth sliding contact (74) are **coaxial.**

2. Medical scope (10) according to the preceding claim, further comprising:
a **deserializer** (60) comprising a two-conductor first interface (62) coupled to the two-conductor first interface (52) of the serializer (50) by a pair of conductors (92, 94, 96) and a multi-conductor second interface (64) connected to a camera control unit (18) or to a connector (16) which can be coupled to a camera control unit (18).

3. Medical scope (10) according to one of the preceding claims, further comprising:
an **image sensor unit** (40) including the image sensor (44), the serializer (50), the first sliding contact (71) and the second sliding contact (72);
a **shaft** (20) with a distal end zone (24) including the third sliding contact (73) and the fourth sliding contact (74).

4. Medical scope (10) according to one of the preceding claims, wherein
the first sliding contact (71) and the second sliding contact (72) are part of a **first coaxial plug connector,**
the third sliding contact (73) and the fourth sliding contact (74) are part of a **second coaxial plug connector** (40) complementary to the first coaxial plug connector.

5. Medical scope (10) according to one of the preceding claims, wherein
an **axis of symmetry** (82) of the coaxial sliding contacts (71, 72, 73, 74) is **parallel** to the viewing direction (48) of the image sensor unit (40).

6. Medical scope (10) according to one of the claims 1 through 4, wherein
an **axis of symmetry** (82) of the coaxial sliding contacts (71, 72, 73, 74) is **orthogonal** to the viewing direction (48) of the image sensor unit (40) and **orthogonal** to the longitudinal axis (28) of the distal end zone (24) of the shaft (20).

7. Medical scope (10) according to one of the preceding claims, further comprising
a fifth sliding contact (75) connected to the third sliding contact (73) and a sixth sliding contact (76) connected to the fourth sliding contact (74);
a seventh sliding contact (77) in electrically conductive contact to the fifth sliding contact (75) and an eighth sliding contact (78) in electrically conductive contact to the sixth sliding contact (76),
wherein at least two of the fifth sliding contact (75), the sixth sliding contact (76), the seventh sliding contact (77) and the eighth sliding contact (78) are **coaxial.**

8. Medical scope (10) according to the preceding claim, wherein
an **axis of symmetry** (82) of at least two of the first sliding contact (71), the second sliding contact (72), the third sliding contact (73) and the fourth sliding contact (74) and an **axis of symmetry** (84) of at least two of the fifth sliding contact (75), the sixth sliding contact (76), the seventh sliding contact (77) and the eighth sliding contact (78) **enclose an angle greater than zero.**

9. Medical scope (10) according to one of the preceding claims, wherein
the serializer (50) and the deserializer (60) comply with the **CSI Camera Serial Interface** specification of the **MIPI** Mobile Industry Processor Interface Alliance.

## Patentansprüche

1. **Medizinische Beobachtungsvorrichtung** (10) mit:
einem **Objektiv** (42), das ein Bild eines Objekts erzeugt;
einem **Bildsensor** (44), der das durch das Objektiv (42) erzeugte Bild erfasst und ein Bildsignal liefert, das das erfasste Bild repräsentiert;
einem **Serialisierer** (50), der eine erste, Zweileiter-Schnittstelle (52) und eine zweite, Mehrleiter-Schnittstelle (54), die zweite Mehrleiter-Schnittstelle (54) mit dem Bildsensor (44) verbunden ist;
**dadurch gekennzeichnet, dass** die medizinische Beobachtungsvorrichtung ferner umfasst:
einen ersten **Gleitkontakt** (71) und einen zweiten **Gleitkontakt** (72), die mit der ersten, Zweileiter-Schnittstelle (52) des Serialisierers (50) verbunden sind;
einen dritten **Gleitkontakt** (73), der mit dem ersten **Gleitkontakt** (71) in elektrisch leitfähigem Kontakt steht, und einem vierten Gleitkontakt (74), der mit dem zweiten Gleitkontakt (72) in elektrisch leitfähigem Kontakt steht,
wobei mindestens zwei von dem ersten Gleitkontakte (71), dem zweiten Gleitkontakte (72), dem dritten Gleitkontakte (73) und dem vierten Gleitkontakte (74) **koaxial** sind.

2. Medizinische Beobachtungsvorrichtung (10) gemäß dem vorhergehenden Anspruch, ferner mit:
einen **Deserialisierer** (60) mit einer ersten, Zweileiter-Schnittstelle (62), die durch ein Paar von Leitern (92, 94, 96) mit der ersten, Zweileiter-Schnittstelle (52) des Serialisierers (50) gekoppelt ist, und einer zweiten, Vielleiter-Schnittstelle (64), die mit einer Kamerasteuereinheit (18) oder einem Verbinder (16), der mit einer Kamerasteuereinheit (18) gekoppelt werden kann, verbunden ist.

3. Medizinische Beobachtungsvorrichtung (10) gemäß einem der vorhergehenden Ansprüche, ferner mit:
eine **Bildsensoreinheit** (40) mit dem Bildsensor (44), dem Serialisierer (50), dem ersten Gleitkontakt (71) und dem zweiten Gleitkontakt (72);
einen **Schaft** (20) mit einem distalen Endbereich (24), der den dritten Gleitkontakt (73) und den vierten Gleitkontakt (74) umfasst.

4. Medizinische Beobachtungsvorrichtung (10) gemäß einem der vorhergehenden Ansprüche, bei der
der erste Gleitkontakt (71) und der zweite Gleitkontakt (72) Teil eines **ersten koaxialen Steckverbinders** sind,
der dritte Gleitkontakt (73) und der vierte Gleitkontakt (74) Teil eines **zweiten koaxialen Steckverbinders** (40) sind, der zu dem ersten koaxialen Steckverbinder komplementär ist.

5. Medizinische Beobachtungsvorrichtung (10) gemäß einem der vorhergehenden Ansprüche, bei der
eine **Symmetrieachse** (82) der koaxialen Gleitkontakte (71, 72, 73, 74) **parallel** zu der Blickrichtung (48) der Bildsensoreinheit (40) verläuft.

6. Medizinische Beobachtungsvorrichtung (10) gemäß einem der Ansprüche 1 bis 4, bei der
eine **Symmetrieachse** (82) der koaxialen Gleitkontakte (71, 72, 73, 74) **orthogonal** zu der Blickrichtung (48) der Bildsensoreinheit (40) und **orthogonal** zu der Längsachse (28) des distalen Endbereichs (24) des Schafts (20) ist.

7. Medizinische Beobachtungsvorrichtung (10) gemäß einem der vorhergehenden Ansprüche, ferner mit
einem fünften Gleitkontakt (75), der mit dem dritten Gleitkontakt (73) verbunden ist, und einem sechsten Gleitkontakt (76), der mit dem vierten Gleitkontakt (74) verbunden ist;
einen siebten Gleitkontakt (77), der in elektrisch leitfähigem Kontakt mit dem fünften Gleitkontakt (75) steht, und einen achten Gleitkontakt (78), der in elektrisch leitfähigem Kontakt mit dem sechsten Gleitkontakt (76) steht,
wobei mindestens zwei von dem fünften Gleitkontakt (75), dem sechsten Gleitkontakt (76), dem siebten Gleitkontakt (77) und dem achten Gleitkontakt (78) **koaxial** sind.

8. Medizinische Beobachtungsvorrichtung (10) gemäß dem vorhergehenden Anspruch, bei der
eine **Symmetrieachse** (82) von mindestens zwei von dem ersten Gleitkontakt (71), dem zweiten Gleitkontakt (72), dem dritten Gleitkontakt (73) und dem vierten Gleitkontakt (74) und eine **Symmetrieachse** (84) von mindestens zwei von dem fünften Gleitkontakt (75), dem sechsten Gleitkontakt (76), dem siebten Gleitkontakt (77) und dem achten Gleitkontakt (78) einen **Winkel größer als Null einschließen.**

9. Medizinische Beobachtungsvorrichtung (10) gemäß einem der vorangehenden Ansprüche, bei der
der Serialisierer (50) und der Deserialisierer (60) der **CSI**-Spezifikation **(Camera Serial Interface)** der **MIPI** Mobile Industry Processor Interface Alliance entsprechen.

## Revendications

1. **Appareil d'examen médical** (10) comprenant :
un **objectif** (42) produisant une image d'un objet ;
un **capteur d'image** (44) capturant l'image produite par l'objectif (42) et fournissant un signal d'image représentant l'image capturée ;
un **convertisseur parallèle-série** (50) comprenant une première interface à deux conducteurs (52) et une seconde interface à plusieurs conducteurs (54), ladite seconde interface à plusieurs conducteurs (54) étant connectée au capteur d'image (44) ;
**caractérisé en ce que** l'appareil d'examen médical comprend en outre :
un premier **contact coulissant** (71) et un deuxième **contact coulissant** (72) connectés à la première interface à deux conducteurs (52) du convertisseur parallèle-série (50) ;
un troisième **contact coulissant** (73) en contact électroconducteur avec le premier contact coulissant (71) et un quatrième **contact coulissant** (74) en contact électroconducteur avec le deuxième contact coulissant (72),
dans lequel au moins deux du premier contact coulissant (71), du deuxième contact coulissant (72), du troisième contact coulissant (73) et du quatrième contact coulissant (74) sont **coaxiaux.**

2. Appareil d'examen médical (10) selon la revendication précédente, comprenant en outre :
un **convertisseur série-parallèle** (60) comprenant une première interface à deux conducteurs (62) couplée à la première interface à deux conducteurs (52) du convertisseur parallèle-série (50) par une paire de conducteurs (92, 94, 96) et une seconde interface à plusieurs conducteurs (64) connectée à une unité de commande de caméra (18) ou à un connecteur (16) qui peut être couplé à une unité de commande de caméra (18).

3. Appareil d'examen médical (10) selon l'une des revendications précédentes, comprenant en outre :
une **unité de capteur d'image** (40) comportant le capteur d'image (44), le convertisseur parallèle-série (50), le premier contact coulissant (71) et le deuxième contact coulissant (72) ;
une tige (20) dotée d'une zone d'extrémité distale (24) comportant le troisième contact coulissant (73) et le quatrième contact coulissant (74).

4. Appareil d'examen médical (10) selon l'une des revendications précédentes, dans lequel
le premier contact coulissant (71) et le deuxième contact coulissant (72) font partie d'un **premier connecteur coaxial à fiche,**
le troisième contact coulissant (73) et le quatrième contact coulissant (74) font partie d'un **second connecteur coaxial à fiche** (40) complémentaire du premier connecteur coaxial à fiche.

5. Appareil d'examen médical (10) selon l'une des revendications précédentes, dans lequel
un axe de symétrie (82) des contacts coulissants coaxiaux (71, 72, 73, 74) est **parallèle** à la direction de visualisation (48) de l'unité de capteur d'image (40).

6. Appareil d'examen médical (10) selon l'une des revendications 1 à 4, dans lequel
un **axe de symétrie** (82) des contacts coulissants coaxiaux (71, 72, 73, 74) est **orthogonal** à la direction de visualisation (48) de l'unité de capteur d'image (40) et **orthogonal** à l'axe longitudinal (28) de la zone d'extrémité distale (24) de la tige (20).

7. Appareil d'examen médical (10) selon l'une des revendications précédentes, comprenant en outre :
un cinquième contact coulissant (75) connecté au troisième contact coulissant (73) et un sixième contact coulissant (76) connecté au quatrième contact coulissant (74) ;
un septième contact coulissant (77) en contact électroconducteur avec le cinquième contact coulissant (75) et un huitième contact coulissant (78) en contact électroconducteur avec le sixième contact coulissant (76),
dans lequel au moins deux du cinquième contact coulissant (75), du sixième contact coulissant (76), du septième contact coulissant (77) et du huitième contact coulissant (78) sont coaxiaux.

8. Appareil d'examen médical (10) selon la revendication précédente, dans lequel
un **axe de symétrie** (82) d'au moins deux du premier contact coulissant (71), du deuxième contact coulissant (72), du troisième contact coulissant (73) et du quatrième contact coulissant (74) et un **axe de symétrie** (84) d'au moins deux du cinquième contact coulissant (75), du sixième contact coulissant (76), du septième contact coulissant (77) et du huitième contact coulissant (78) **forment un angle supérieur à zéro.**

9. Appareil d'examen médical (10) selon l'une des revendications précédentes, dans lequel le convertisseur parallèle-série (50) et le convertisseur série-parallèle (60) sont conformes à la spécification **CSI, Camera Serial interface** (interface série pour caméra), de la **MIPI,** Mobile Industry Processor Interface Alliance.
